(19) Europäisches Patentamt
European Patent Office
Office européen des brevets

(11) EP 1 654 357 B1

(12) **EUROPÄISCHE PATENTSCHRIFT**

(45) Veröffentlichungstag und Bekanntmachung des Hinweises auf die Patenterteilung:
**06.10.2010 Patentblatt 2010/40**

(21) Anmeldenummer: **04741371.1**

(22) Anmeldetag: **05.08.2004**

(51) Int Cl.:
***C12N 11/02*** *(2006.01)*

(86) Internationale Anmeldenummer:
**PCT/EP2004/008770**

(87) Internationale Veröffentlichungsnummer:
**WO 2005/017142 (24.02.2005 Gazette 2005/08)**

(54) **VERWENDUNG VON PIT-EMULSIONEN IN ENZYMATISCHEN REAKTIONEN**
USE OF PIT EMULSIONS IN ENZYMATIC REACTIONS
UTILISATION D'EMULSIONS PIT DANS DES REACTIONS ENZYMATIQUES

(84) Benannte Vertragsstaaten:
**DE ES FR GB IT**

(30) Priorität: **14.08.2003 DE 10337451**

(43) Veröffentlichungstag der Anmeldung:
**10.05.2006 Patentblatt 2006/19**

(73) Patentinhaber: **Cognis IP Management GmbH
40589 Düsseldorf (DE)**

(72) Erfinder:
- **WEISS, Albrecht
40764 Langenfeld (DE)**
- **DUBREUCQ, Eric
F-34000 Montpellier (FR)**
- **MÜLLER, Michael
40789 Monheim (DE)**
- **MOULIN, Guy
F-34980 Montferrier-sur-Lez (FR)**

(56) Entgegenhaltungen:
**WO-A-91/00918 US-A- 4 839 287**

- **B ORLICH, R SCHOMÄCKER: "Candida Rugosa lipase reactions in nonionic w/o-microemulsion with a technical surfactant" ENZYME AND MICROBIAL TECHNOLOGY, Bd. 2, Nr. 1, 2. Januar 2001 (2001-01-02), Seiten 42-48, XP002307261 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 654 357 B1

**Beschreibung**

**Gebiet der Erfindung**

Die vorliegende Erfindung betrifft die Verwendung von Emulsionen, die nach dem PIT-Verfahren hergestellt werden, als Reaktionsmedium für enzymatisch katalysierte Reaktionen.

**Stand der Technik**

In der chemischen und biochemischen Synthese werden vermehrt Enzyme als Katalysatoren eingesetzt. So werden in vielen Fällen aufgrund der oft milderen Reaktionsbedingungen bereits in großtechnischen Verfahren Hydrolasen, speziell Lipasen (EC 3.1.1.3) zur Fettspaltung eingesetzt.

Geeignete enzymatische Verfahren zur Veresterung oder Umesterung sind beispielsweise beschrieben in K. Drauz und H. Waldmann, Enzyme Catalysis in Organic Synthesis, VCH-Verlag, Weinheim 1975.

Bekannt ist, dass Transesterifikation in wasserfreien oder wasserarmen Medien durch Lipasen katalysiert werden. Ist im Reaktionssystem von Estern, Alkohol und Lipasen auch Wasser vorhanden, so setzt normalerweise eine Abspaltung von gebundenen Säuren zu freien Säuren ein. Da verschiedene Lipasen auch die Bildung von Estern aus freien Fettsäuren und Alkoholen katalysieren, werden im Endeffekt in den überwiegenden Fällen eine Transesterifikationsreaktion mit einer Säurezwischenstufe neben einer direkten Transesterifikation durchgeführt. Für viele technische Prozesse ist allerdings von großem Nachteil, dass freie Säuren im System gebildet werden. Teilweise verhindert der Wassergehalt eine technisch und kommerziell akzeptable Umsetzung (Ausbildung eines unvorteilhaften thermodynamischen Gleichgewichts). Aufwendige technische Anlagen (Wasserentfernung über z.B. azeotrope Destillation, Membrantrennverfahren, Vakuumdestillation) müssen zur Erzielung befriedigender Ausbeuten eingesetzt werden.

Der Nachteil bei enzymatisch katalysierten Reaktionen liegt oft in der Verfügbarkeit und Stabilität der am Prozess beteiligten funktionellen Proteine. Aus dem Stand der Technik sind bereits Enzyme bekannt, die durch Immobilisierung beispielsweise durch Mikroeinkapselung stabilisiert vorliegen und mehrfach Verwendung finden können.

Die Umsetzung von hydrophoben Verbindungen kann bereits durch den Einsatz von Wasser in Öl (W/O) Mikroemulsionen durchgeführt werden, wie es von Orlich und Schomaeker in Enzyme Microb. Technol.; 2001; 28; 1; 42-48 für die Lipase aus *Candida rugosa* beschrieben wird. Sehr kritisch für die erfolgreiche Umsetzung ist jedoch die Konzentration an Wasser in der Lösung sowie die Zusammensetzung der Komponenten der W/O-Mikroemulsion.

Die Aufgabe der vorliegenden Erfindung hat somit darin bestanden, ein System für enzymatisch katalysierte Reaktionen zur Verfügung zu stellen, bei dem die Substratkonzentrationen variiert werden können wobei jedoch die Grenzflächen und damit die Konzentrationen an Öl und Wasser soweit konstant bleiben, dass sie keinen großen Einfluss auf die Reaktion und auf die Aktivität des Enzyms haben. Des weiteren sollten diese Systeme kostengünstig und recyclingfähig sein.

**Beschreibung der Erfindung**

Gegenstand der Erfindung ist die Verwendung von O/W-Emulsionen als Reaktionsmedium für enzymatisch katalysierte Reaktionen, enthaltend mindesten Wasser, Emulgatoren sowie eine Ölphase, wobei die Emulsion nach dem PIT-Verfahren hergestellt wird und eine Tröpfchengröße von 50 bis 400 nm aufweist.

Überraschenderweise wurde gefunden, dass O/W-Emulsionen die nach dem PIT-Verfahren hergestellt wurden, die eingangs geschilderten Anforderungen in ausgezeichneter Weise erfiillen.

PIT-Emulsionen

Emulsionen sind disperse Zubereitungen aus mindestens zwei nicht ineinander löslichen Flüssigkeiten von denen eine wässrig ist. Zur Homogenisierung nicht mischbarer Öl/Wasser-Phasen durch Emulgierung werden Emulgatoren beziehungsweise Emulgatorsysteme eingesetzt. Ohne Einfluss von stabilisierenden Emulgatoren würden sich die Phasen aufgrund ihrer unterschiedlichen Polaritäten wieder trennen. Die amphiphilen Emulgatoren sitzen an den Grenzflächen zwischen den fein verteilten Tröpfchen und der kohärenten Phase und verhindern durch sterische oder elektrostatische Abschirmung ihr Zusammenfließen (= Koaleszenz). Emulgatoren sind Verbindungen, die in ihrer Molekülstruktur hydrophile und lipophile Bausteine miteinander verbinden. Auswahl und Ausmaß der jeweiligen Bausteine im betroffenen Emulgatormolekül beziehungsweise Emulgatorsystem werden häufig durch den HLB-(number)Wert gekennzeichnet, der eine Aussage zur Hydrophilic-Lipophilic-Balance macht. Dabei gilt üblicherweise: Emulgatoren beziehungsweise Emulgatorsysteme mit vergleichsweise stark hydrophilen Anteilen führen zu hohen HLB-Werten und in ihrer praktischen Anwendung in der Regel zu den Wasser-basierten O/W-Emulsionen mit disperser Ölphase. Emulgatoren beziehungsweise Emulgatorsysteme mit vergleichsweise stark lipophilen Anteilen führen zu vergleichsweise niedrigeren HLB-Werten und damit zur W/O-Invertemulsion mit geschlossener Ölphase und disperser Wasserphase.

Es ist bekannt, daß Öl-in-Wasser-Emulsionen (O/W), die mit nichtionogenen Emulgatoren hergestellt und stabilisiert sind, bei Erwärmen eine in der Regel reversible Phaseninversion erfahren können, d.h., dass innerhalb eines bestimmten Temperaturintervalls ein Wechsel des Emulsionstyps von O/W zu W/O (Wasser-in-Öl-Emulsion) erfolgt. Da dabei das Öl zur äußeren, kontinuierlichen Phase wird, sinkt die Leitfähigkeit der Emulsion auf Null. Der Mittelwert der Temperaturen zwischen maximaler und gerade auf Null gefallener Leitfähigkeit der Emulsion bei Temperaturerhöhung wird Phaseninversionstemperatur (PIT) und die auf diese Weise hergestellten Emulsionen werden PIT-Emulsionen genannt.

Es ist auch bekannt, dass die Lage der PIT von vielen Faktoren abhängig ist, zum Beispiel von der Art und des Phasenvolumens der Ölkomponente, von der Hydrophilie und der Struktur der Emulgatoren und der Zusammensetzung des Emulgatorsystems.

Wesentlich für die Feinteiligkeit der PIT-Emulsion ist deren Herstellungsverfahren. In der Regel werden die Wasser- und Ölphase mit den Emulgatoren vermischt und danach auf eine Temperatur oberhalb der PIT erwärmt. Die Leitfähigkeit muß dabei auf Null fallen. Anschließend wird die Emulsion wieder auf die Ausgangstemperatur abgekühlt (in der Regel Raumtemperatur, ca. 20°C). Dabei erfolgt erst durch Überschreiten und das anschließende Unterschreiten der PIT die Ausbildung der erfindungsgemäß verwendeten Emulsion.

Es ist bekannt, dass nur solche PIT-Emulsionen besonders feinteilig sind, welche bei der Phaseninversion eine Mikroemulsionsphase mit niedriger Grenzflächenspannung zwischen Öl und Wasser oder eine lamellare flüssigkristalline Phase ausbilden. Der entscheidende Schritt ist dabei immer die Rückinvertierung bei Abkühlung.

Die erfindungsgemäßen Emulsionen zeichnen sich insbesondere durch ihre Feinteiligkeit aus. Die Tröpfchengröße beträgt 50 bis 400 nm. Vorzugsweise liegt die Tröpfchengröße im Bereich von 70 bis 300 nm, insbesondere im Bereich von 80 bis 250 nm und besonders bevorzugt im Bereich von 90 bis 160 nm. Bei den Tröpfchengrößen wird eine Verteilung nach Gauss angenommen. Die Messung erfolgt beispielsweise durch Lichtstreuung oder Absorption.

Diese feinverteilten Emulsionen behalten ihre Homogenität durch die Brownsche Molekularbewegung. Diese Brownsche Molekularbewegung ist eine thermische Zufallsbewegung von Teilchen < 5 μm. Sie ist die treibende Kraft der Diffusion und behindert sowohl die Sedimentation als auch das Aufrahmen (Flotation). Ein großer Vorteil besteht darin, dass energieaufwendige Rührvorgänge vermindert werden können. Sie führt zu einer verbesserten Diffusion von Substrat und Enzym und zu verminderten Energiekosten.

Die Substratkonzentrationen können variiert werden ohne dass die Tropfengröße verändert werden muss. Eine hohe Substratkonzentration kann erreicht werden, ohne das eine Koaleszens der Tropfen auftritt. Die geringe Oberflächenspannung erhöht die Transferrate der Moleküle an der Öl/Wasser Grenzfläche. Die hohe Reproduzierbarkeit und Stabilität der PIT-Emulsionen ermöglicht biochemische Studien an Enzymen und deren Reaktivität sowie die Möglichkeit bereits bekannte Reaktionsbedingungen und Aktivitäten für Enzyme noch weiter zu optimieren.

Die PIT-Emulsionen enthalten, neben Wasser noch eine **Ölphase**, die Verbindungen aus der Gruppe der Fettsäurealkylester a) oder der nativen pflanzlichen Öle und deren Derivate b) enthält. Es handelt sich bei den Gruppen a) und b) um hydrophobe, in Wasser nicht oder nur sehr gering lösliche Verbindungen, die bevorzugt die Ausgangsstoffe, also Substrate für die durch enzymatische Katalyse gewünschten Produkte darstellen können aber auch als Hilfsstoffe eingesetzt werden können.

Geeignete Ester der Gruppe a) leiten sich insbesondere ab von gesättigten, ungesättigten, linearen oder verzweigten Fettsäuren mit insgesamt 7 bis 23 Kohlenstoffatomen. Es handelt sich also um Verbindungen der Formel (I)

$$R^1\text{-COO-}R^2 \qquad (I)$$

wobei R1 für einen Alkylrest mit 6 bis 22 C-Atomen steht und R2 ein Alkylrest mit 1 bis 4 C-Atomen ist, wobei Methyl- und Ethylreste besonders bevorzugt sind. Am vorteilhaftesten ist der Einsatz von Methylestern. Die Methylester der Formel (I) können auf übliche Weise, z.B. durch Umesterung von Triglyceriden mit Methanol und anschließender Destillation, erhalten werden. Geeignete Fettsäuren sind die Capron-, Heptan-, Capryl-, Perlagon-, Caprin-, Undecan-, Laurin-, Tridecan-, Myristin-, Pentadecan-, Palmitin-, Heptadecan-, Stearin-, Nonadecan-, Arachin- und Behensäure. Ungesättigte Vertreter sind beispielsweise Lauroelein-, Myristolein-, Palmitolein-, Petroselaidin-, Öl-, Elaidin-, Ricinol-, Linol-, konjugierte Linolsäure (CLA), insbesondere die cis9, trans11-CLA oder die trans10,cis12-CLA, Linolaidin-, Linolen-, konjugierte Linolsäure Gadolein-, Arachidon und Erucasäure. Auch Mischungen der Methylester und/oder Ethylester dieser Säuren sind geeignet. Besonders bevorzugt ist die Verwendung solcher PIT-Emulsionen, die Methylester und/oder Ethylester aus der Gruppe Methyloleat, Methylpamitat, Methylstearat, Methylpelargonat, Ethyloleat, Ethylpamitat, Ethylstearat und/oder Ethylpelargonat enthalten. Es können aber auch Methylester und/oder Ethylester auf Basis natürlicher Fettsäuremischungen eingesetzt werden, wie sie beispielsweise aus Lein-, Kokos-, Palm-, Palmkern-, Oliven- , Ricinus-, Rüb-, Soja- oder Sonnenblumenölen (bei Raps- und Sonnenblumenöl jeweils neue und alte Züchtungen) erhalten werden.

Geeignete Verbindungen der Gruppe b) sind native Öle pflanzlichen Ursprungs. Es handelt sich dabei im

Wesentlichen um Triglyceride und Triglyceridmischungen, wobei das Glycerin mit längerkettigen Fettsäuren jeweils vollständig verestert ist. Besonders geeignete pflanzliche Öle sind ausgewählt aus der Gruppe Erdnuß-, Kokos- und/ oder Sonnenblumenöl.

Wichtige Bestandteile der erfindungsgemäß verwendeten PIT-Emulsionen sind die eingesetzten **Emulgatoren** bzw. Emulgatorensysteme. Vorzugsweise werden als Emulgatoren nichtionische Emulgatoren, insbesondere ethoxylierte Fettalkohole und Fettsäuren eingesetzt. Zur Ausbildung von PIT-Emulsionen ist es vorteilhaft ein zweikomponentiges Emulgatorsystem, enthaltend einen hydrophilen Emulgator (A) und einen hydrophoben Coemulgator (B) einzusetzen. Als hydrophile nichtionische Emulgatoren (A) eignen sich Stoffe, die einen HLB-Wert von etwa 8 bis 18 aufweisen. Unter dem HLB-Wert (Hydrophil-Lipophil-Balance) soll ein Wert verstanden werden, der gemäß

$$HLB = (100\text{-}L) / 5$$

errechnet werden kann, wobei L der Gewichtsanteil der lipophilen Gruppen, d.h. der Fettalkyl- oder Fettacylgruppen in Prozent in den Ethylenoxidanlagerungsprodukten ist.

Fettalkoholethoxylate im Sinne der erfindungsgemäßen Lehre folgen der allgemeinen Formel (II)

$$R^3\text{-O-}(CH_2CH_2O)_n\text{-H} \qquad (II)$$

wobei $R^3$ für einen linearen oder verzweigten, gesättigten oder ungesättigten Alkylrest mit 6 bis 24 Kohlenstoffatomen steht und n eine Zahl von 1 bis 50 bedeutet. Besonders bevorzugt sind solche Verbindungen der Formel (II), in der n für eine Zahl von 1 bis 35 und insbesondere von 1 bis 15 steht. Besonders bevorzugt sind weiterhin solche Verbindungen der Formel (II), in der $R^3$ für einen Alkylrest mit 16 bis 22 Kohlenstoffatomen steht.

Die Verbindungen der Formel (II) werden in an sich bekannter Weise durch Umsetzung von Fettalkoholen mit Ethylenoxid unter Druck, ggf. in Gegenwart saurer oder basischer Katalysatoren erhalten. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Fettsäureethoxylate, die ebenfalls als Emulgatorkomponente (A) in Betracht kommen, folgen vorzugsweise der Formel (III),

$$R^4CO_2(CH_2CH_2O)_mH \qquad (III)$$

in der $R^4$ für einen linearen oder verzweigten Alkylrest mit 12 bis 22 Kohlenstoffatomen und m für Zahlen von 5 bis 50 und vorzugsweise 12 bis 35 steht. Typische Beispiele sind Anlagerungsprodukte von 10 bis 30 Mol Ethylenoxid an Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen oder bei der Reduktion von Aldehyden aus der Roelenschen Oxosynthese anfallen. Vorzugsweise werden Anlagerungsprodukte von 10 bis 30 Mol Ethylenoxid an Fettsäuren mit 16 bis 18 Kohlenstoffatomen eingesetzt.

Partialglyceride, die als Emulgatorkomponente (B) in Betracht kommen, folgen vorzugsweise der Formel (IV),

$$\begin{array}{l} CH_2O(CH_2CH_2O)_x\text{-}COR^5 \\ | \\ CH\text{-}O(CH_2CH_2O)_yH \qquad\qquad (IV) \\ | \\ CH_2O(CH_2CH_2O)_z\text{-}H \end{array}$$

in der $COR^5$ für einen linearen oder verzweigten Acylrest mit 12 bis 22 Kohlenstoffatomen und x, y und z in Summe für 0 oder für Zahlen von 1 bis 50, vorzugsweise 15 bis 35 steht. Typische Beispiele für im Sinne der Erfindung geeignete Partialglyceride sind Laurinsäuremonoglycerid, Kokosfettsäuremonoglycerid, Palmitinsäuremonoglycerid, Stearinsäuremonoglycerid, Isostearinsäuremonoglycerid, Ölsäuremonoglycerid, konjugierte Linolsäuremonoglyceride und Talgfettsäuremonoglycerid sowie deren Addukte mit 5 bis 50 und vorzugsweise 20 bis 30 Mol Ethylenoxid. Vorzugsweise werden Monoglyceride bzw. technische Mono/Diglyceridgemische mit überwiegendem Monoglyceridanteil der Formel (IV) eingesetzt, in der $COR^5$ für einen linearen Acylrest mit 16 bis 18 Kohlenstoffatomen steht.

Üblicherweise werden Emulgatormischungen eingesetzt, die die Komponenten (A) und (B) im Gewichtsverhältnis 10 : 90 bis 90 : 10, vorzugsweise 25 : 75 bis 75 : 25 und insbesondere 40 : 60 bis 60 : 40 enthalten.

Als weitere geeignete Emulgatoren kommen beispielsweise nichtionogene Tenside aus einer der folgenden Gruppen in Frage:

- Anlagerungsprodukte von 2 bis 30 Mol Ethylenoxid vorzugsweise 4 bis 10 Mol Ethylenoxid und/ oder 0 bis 5 Mol Propylenoxid an lineare Fettalkohole mit 8 bis 22 C-Atomen;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte mit 1 bis 30 Mol Ethylenoxid;
- Alkylmono- und -oligoglycoside mit 8 bis 22 Kohlenstoffatomen im Alkylrest und deren ethoxylierte Analoga;
- Anlagerungsprodukte von 15 bis 60 Mol Ethylenoxid an Ricinusöl und/oder gehärtetes Ricinusöl;
- Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
- Anlagerungsprodukte von 2 bis 15 Mol Ethylenoxid an Ricinusöl und/oder gehärtetes Ricinusöl;
- Partialester auf Basis linearer, verzweigter, ungesättigter bzw. gesättigter $C_{12/22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
- Wollwachsalkohole;
- Polyalkylenglycole.

Die Anlagerungsprodukte von Ethylenoxid und/oder von Propylenoxid an Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Zur Auswahl geeigneter Emulgatorsysteme kann es zweckmäßig sein, rechnerisch die Ermittlung der PIT des jeweiligen Systems durchzuführen. Insbesondere gilt das aber auch für potentielle Optimierungen in der Auswahl der Emulgatoren beziehungsweise Emulgatorsysteme und ihrer Anpassung an die durch sonstige Überlegungen zum technischen Handeln vorgegebene Auswahl und Abmischung von wäßriger Phase einerseits und Typ der Ölphase andererseits. Entsprechendes Fachwissen ist aus an sich ganz anderen Bereichen, insbesondere aus dem Bereich der Kosmetika-Herstellung, entwickelt worden. Verwiesen wird insbesondere auf die Veröffentlichung TH.Förster, W.von Rybinski, H.Tesmann und A.Wadle "Calculation of optimum emulsifier mixtures for phase inversion emulsification", in International Journal of Cosmetic Science 16, 84-92 (1994). Dargestellt ist hier im einzelnen wie auf rechnerischem Weg für vorgegebene 3-Komponenten-Systeme aus einer Ölphase, einer Wasserphase und einem Emulgator auf der Basis des für die Ölphase charakteristischen EACN-Wertes (equivalent alkane carbon number) der Temperaturbereich der Phaseninversion (PIT) über die CAPICO-Methode (calculation of phase inversion in concentrates) errechnet werden kann. Diese Veröffentlichung Förster et al. bezieht insbesondere wiederum wesentliche Literatur für den hier angeschnittenen Themenkomplex ein, die im Zusammenhang mit der Offenbarung dieser Veröffentlichung Förster et al. zu sehen ist. Im einzelnen wird dann anhand zahlreicher Beispiele dargestellt, wie mittels der CAPICO-Methode im Rahmen des EACN-Konzepts die Auswahl und Optimierung der Emulgatoren/Emulgatorsysteme zur optimalen Einstellung vorgegebener Werte für den Temperaturbereich der Phaseninversion zugänglich wird.

Die erfindungsgemäß verwendeten PIT-Emulsionen enthalten vorzugsweise von 20 bis 90 Gew.-% Wasser, insbesondere von 30 bis 80 Gew.-% und ganz besonders bevorzugt von 30 bis 60 Gew.-%. Der Rest auf 100 Gew.-% entfällt auf die Ölphase sowie Emulgatoren und ggf. weitere Hilfs- und Zusatzstoffe. Die Ölphase selbst ist vorzugsweise in Mengen von 10 bis 80 Gew.-%, insbesondere von 40 bis 70 Gew.-% enthalten. Dabei enthält die Ölphase vorzugsweise ausschließlich die Komponenten a) oder b) bzw. Mischungen dieser Komponenten. Die Emulgatoren, bzw. Emulgatorensysteme sind vorzugsweise in Mengen von 1 bis 25 Gew.-%, insbesondere in Mengen von 5 bis 20 und besonders bevorzugt in Mengen von 5 bis 15 Gew.-% enthalten. Die erfindungsgemäß verwendeten Emulsionen weisen vorzugsweise Phaseninversionstemperaturen im Bereich von 20 bis 95 °C und insbesondere von 30 bis 95 °C auf.

Die erfindungsgemäß verwendeten Enzyme stellen Enzyme dar, die grenzflächenaktiv sind. Vorzugsweise werden Enzyme aus der Gruppe der Hydrolasen und/oder Acyltransferasen eingesetzt die entweder allein oder in Kombination mit mehreren Enzymen eingesetzt werden können.

Besonders bevorzugt sind Hydrolasen ausgewählt aus der Gruppe, die gebildet wird von Esterasen, Phospholipasen, Lipasen und Lipasen/Acyltransferasen. Bei letzten handelt es sich um Enzyme, die grenzflächenaktiv sind und Reaktionen katalysieren, die charakteristisch für Lipasen sind. Es wurde gefunden, dass diese Polypeptide in der Lage

sind, Transesterifizierungen in Gegenwart kurzkettiger Alkohole zu katalysieren bei einem Wassergehalt im Reaktionsgemisch, der einer Wasser-Aktivität größer 0,8 entspricht. Bei diesem Gehalt an Wasser würde eine herkömmliche Lipase in Abhängigkeit von der Alkoholkomponente überwiegend die Hydrolyse der Ester katalysieren. Es handelt sich also um Enzyme, welche sowohl charakteristische Merkmale für Lipasen als auch für Acyltransferasen zeigen. Bei dem erfindungsgemäßen, natürlich vorkommendem Enzym handelt es sich aufgrund von Sequenzhomologien zu bislang bekannten Enzymen wie beispielsweise Lipase aus *Candida parapsilosis* um eine Lipase und aufgrund seiner enzymatischen Aktivität um eine Acyltransferase.

Typische Beispiele für geeignete Enzyme, die jedoch nicht einschränkend sein sollen, sind Lipasen und/oder Lipasen/Acyltransferasen von Organismen die ausgewählt sind aus der Gruppe, die gebildet wird von *Alcaligenes, Aspergillus niger, Aspergillus oryzea, Aeromonas aerophila, Bacillus species, Candida albicans, Candida antarctica (Trychosporon oryzae, Pseudozyma antarctica), Candida antarctica, Candida cylindracea, Candida glabrata, Candida maltosa, Candida parapsilosis, Candida lipolytica, Candida tropicalis, Candida viswanathii, Chromobacterium viscosum, Fusarium solani, Geotrichum candidum, Issatchenkia orientalis (Candida krusei), Kluyveromyces marxianus (C. kefyr, C. pseudotropicalis), Mucor javanicus, Penicilium camenberti, Penicilium roqueforti, Pichia guilliermondii (Candida guilliermondii), Porcine pancreas, Pseudomonas cepacia, Pseudomonas fluorescens, Rhizomucor miehei, Rhizopus arrhizus, Rhizopus oryzae, Rhizopus niveus, Rhizopus javanicus* und *Thermomyces lanugenosus* sowie deren Gemischen. Bevorzugt, weil besonders aktiv, sind Lipasen und Lipasen/Acyltransferasen aus den Organismen *Alcaligenes, Candida, Chromobacterium, Rhizomucor, Pseudomonas, Rhizopus und Thermomyces* insbesonderer um Enzyme aus *Candida parapsilosis, Pichia guilliermondii (Candida guilliermondii)* oder *Candida antarctica.*

Die erfindungsgemäß einzusetzenden Enzyme können in unterschiedlichen Formen eingesetzt werden. Prinzipiell sind alle dem Fachmann gebräuchlichen Darreichungsformen von Enzymen anwendbar. Unter die Definition "Enzym" fallen erfindungsgemäß auch die Begriffe Protein und Enzym-Protein. Erfindungsgemäß ist sowohl das Enzymprotein als auch das Gesamtprotein welches in einem Teil der Proteinsequenz die Funktion des erfindungsgemäßen Proteins beinhaltet anzuwenden. Vorzugsweise werden die Enzyme in reiner Form oder als technisches Enzympräparat entweder immobilisiert auf Trägermaterial und/oder in Lösung, insbesondere in wässriger Lösung eingesetzt und in sogenannten "repeated batches" wiederverwendet. Ebenfalls bevorzugt sind kristallisierte Enzyme, sogenannte CLEC's, beispielweise zu beziehen von der Firma Altus. Der Anteil an aktiven Enzym in den jeweiligen technischen Enzympräparaten variiert von Hersteller zu Hersteller. Der Anteil liegt jedoch im Mittel zwischen 1 und 10 % aktiven Enzym.

Die erfindungsgemäß einzusetzenden Enzyme werden in einer weiteren Ausführungsform der Erfindung eingesetzt in einer Menge von 0,001 bis 20 Gew.-% berechnet als reines Enzym oder als Enzympräparat, bezogen auf die Gesamtmenge an eingesetzter Ölphase. Im Besonderen liegt die einzusetzende Menge bei 0,002 bis 1 Gew.-% und besonders bevorzugt bei 0,002 bis 0,2 Gew.-%.

Im Sinne der Erfindung handelt es sich bei den enzymatisch katalysierten Reaktionen bevorzugt um Hydrolysen, Umesterungen oder Veresterungen, wobei die Umesterungen besonders bevorzugt sind.

Im Sinne der Erfindung werden durch die enzymatisch katalysierten Reaktionen unter Verwendung der erfindungsgemäßen PIT-Emulsion kosmetische und/oder pharmazeutische Produkte und/oder Feinchemikalien hergestellt. Im besonderen werden Carotinoide, sterolhaltige Ölkomponenten und/oder Vitamin E hergestellt.

Die erfmdungsgemäßen O/W-Emulsionen, enthaltend mindesten Wasser, Emulgatoren sowie eine Ölphase und hergestellt nach dem PIT-Verfahren eignen sich hervorragend für die Verwendung als Reaktionsmedium für enzymatisch katalysierte Reaktionen. Hieraus lässt sich direkt ein weiterer Gegenstand der Erfindung ableiten. Es handelt sich um ein Verfahren zur enzymatisch katalysierten Hydrolyse, Umesterung oder Veresterung von Fettsäurealkylestern und/oder Triglyceriden, bei dem O/W-Emulsionen als Reaktionsmedium eingesetzt werden, die nach dem PIT-Verfahren hergestellt werden. Die für das erfindungsgemäße Verfahren eingesetzten Emulsionen entsprechen in ihren Bestandteilen, Bedingungen und näheren Ausführungsformen den Emulsionen, die bereits für die Verwendung dieser O/W-Emulsionen näher beschrieben wurden. Nach dem erfindungsgemäßen Verfahren werden bevorzugt kosmetische und/oder pharmazeutische Produkte und/oder Feinchemikalien hergestellt. Im besonderen werden Carotinoide, sterolhaltige Ölkomponenten und/oder Vitamin E hergestellt. Bei diesem Verfahren kann man es sich zu nutze mache, dass die nicht wasserlöslichen Substrate, in der Ölphase der PIT-Emulsion löslich werden und so der enzymatisch katalysierten Reaktion zugänglich sind.

Die PIT-Emulsionen enthaltend das Substrat werden erfindungsgemäß dem Reaktionsgefäß welches die Lipase bzw. Lipase/Acyltransferase immobilisiert oder nicht immobilisiert sowie ggf. weitere Hilfs- und Zusatzstoffe enthält, zudosiert. Die Einzelheiten dieses Verfahrens, insbesondere die Menge an Enzym und der zudosierten Emulsion, ergeben sich aus der Art der Enzyme und der gewählten PIT-Emulsion und können vom Fachmann an die spezifischen Gegebenheiten angepasst werden. Durch Erhitzen des Systems kann eine Phasentrennung erreicht werden und der Emulgator in der wässrigen Phase und das Produkt in der Ölphase in einfacher Weise voneinander getrennt werden. Durch die Verwendung von Festbettreaktoren enthaltend das Enzym ist eine Abtrennung und Wiederverwendung des Enzyms gegeben.

Die Feinteiligkeit der Öltröpfchen führt zu einer großen Oberfläche zwischen Öl- und Wasserphase und ermöglicht so einen schnellen Kontakt und eine erhöhte Reaktionsrate zwischen den Enzymen und der die Substrate enthaltenden Ölphase.

In einer besonderen Ausführungsform dieses Verfahrens werden Enzyme eingesetzt, die bereits für die Verwendung der erfindungsgemäßen O/W-Emulsion aufgelistet wurden.

Die erfindungsgemäßen Reaktionsbedingungen der enzymatisch katalysierten Reaktion richten sich nach dem optimalen Reaktionsbereich der ausgewählten Enzyme und nach den verwendeten Emulsionen. Im Besonderen handelt es sich um Bedingungen bei denen unter anderem die Reaktionstemperatur zwischen 15 und 50 °C, bevorzugt eine Temperatur zwischen 20 und 40 °C, insbesondere eine Temperatur von 35 °C gewählt wird.

## Beispiele

Herstellbeispiel H1

Umesterung von Ölsäureethylester mit Methanol oder Hydrolyse von Ölsäureethylester durch Lipase/Acyltransferase und Hydrolyse des eingesetzten Ölsäureesters.

Zur Herstellung der PIT-Emulsion von C18:1 Ölsäureethylester wurde eine Mischung aus 0,25 g Polyoxyethylen-12-Cetylstearylalkohol (Eumulgin® B1) und 0,2 g Polyoxyethylen-6-Cetylalkohol mit 1 g Ölsäureethylester und 3 g ultrareinem Wasser unter Rühren bis auf 95 °C aufgeheizt, bis alle Komponenten geschmolzen waren. Anschließend wurde unter Rühren bis auf Raumtemperatur abgekühlt. Die Phaseninversionstemperatur (PIT) lag bei ungefähr 66 °C. Diese Emulsion (10 μmol/ml) wurde zu einer auf pH 6,5 gepufferten Lösung enthaltend Lipase/Acyltransferase (CPLIP2) und 2,2 mol/l Methanol als Substrat gegeben und die Hydrolyse bzw. die Methanolyse in μmol/min/ml in Abhängigkeit von der Enzymmenge [μg/ml] bestimmt. Die Ergebnisse sind in Tabelle 1 wiedergegeben.

Tabelle 1:

| Enzymmenge [μg/ml] | Hydrolyse [μmol/min/ml] | Methanolyse [μmol/min/ml] |
|---|---|---|
| 1 | 0,001 | 0,005 |
| 2 | 0,003 | 0,009 |
| 5 | 0,015 | 0,025 |
| 10 | 0,038 | 0,050 |
| 15 | 0,088 | 0,066 |

Des weiteren wurde die Aktivität des Enzyms in der PIT-Emulsion mit Ölsäureethylester in Gegenwart von Methanol oder Isopropanol bestimmt. Die Ergebnisse sind in Tabelle 2 und 3 dargestellt.

Tabelle 2: Aktivität des Enzyms [14 μg/ml] bei 45 °C

| Methanol [mol/l] | Hydrolyse [μmol/min/ml] | Methanolyse [μmol/min/ml] |
|---|---|---|
| 0 | 0,089 | 0 |
| 0,5 | 0,037 | 0,024 |
| 1 | 0,024 | 0,034 |
| 2,2 | 0,009 | 0,074 |
| 4 | 0,002 | 0,128 |

Tabelle 3: Aktivität des Enzyms [35 μg/ml] bei 30 °C

| Isopropanol [mol/l] | Hydrolyse [μmol/min/ml] | Isopropanolyse [μmol/min/ml] |
|---|---|---|
| 0 | 0,042 | 0 |
| 0,1 | 0,043 | 0 |
| 0,2 | 0,031 | 0,009 |
| 0,5 | 0,032 | 0,017 |

(fortgesetzt)

| Isopropanol [mol/l] | Hydrolyse [µmol/min/ml] | Isopropanolyse [µmol/min/ml] |
|---|---|---|
| 1,0 | 0,027 | 0,046 |
| 1,25 | 0,023 | 0,066 |
| 1,5 | 0,022 | 0,11 |
| 1,75 | 0,020 | 0,17 |
| 2,0 | 0,009 | 0,19 |
| 2,25 | 0,009 | 0,20 |
| 2,5 | 0,0003 | 0,17 |
| 3,0 | 0 | 0,027 |

Diskussion der Ergebnisse:

Die Ergebnisse zeigen, dass sowohl die Umesterung der Methanolyse als auch die Hydrolyse durchführbar sind in einer PIT Emulsion und das das Enzym die besonderen Fähigkeiten nicht verliert. Es zeigt sich weiterhin, dass eine Methanolyse in Gegenwart von Wasser der PIT-Emulsion gegenüber einer Hydrolyse bevorzugt ist.

Die Umesterung zu Isopropyloleat in dem genannten System ist ebenfalls durchführbar wobei die Aktivität des Enzyms bei 2,25 mol/l Isopropanol ein Maximum anzeigt.

**Patentansprüche**

1. Verwendung von O/W-Emulsionen als Reaktionsmedium für enzymatisch katalysierte Reaktionen, enthaltend mindesten Wasser, Emulgatoren sowie eine Ölphase, **dadurch gekennzeichnet, dass** die Emulsion nach dem PIT-Verfahren hergestellt wird und eine Tröpfchengröße von 50 bis 400 nm aufweist.

2. Verwendung nach Anspruch 1, **dadurch gekennzeichnet, dass** die Ölphase Verbindungen enthält, ausgewählt aus der Gruppe, die gebildet wird von Fettsäurealkylester und Triglyceride.

3. Verwendung nach den Ansprüchen 1 und/oder 2, **dadurch gekennzeichnet, dass** Emulsionen verwendet werden, die Fettsäurealkylester der Formel (I) enthalten

$$R^1\text{-COO-}R^2 \qquad \text{(I)}$$

wobei $R^1$ für einen Alkylrest mit 6 bis 22 C-Atomen steht und $R^2$ einen Alkylrest mit 1 bis 4 C-Atomen bedeutet.

4. Verwendung nach den Ansprüchen 1 bis 3, **dadurch gekennzeichnet, dass** Emulsionen verwendet werden, die die Ölphase in Mengen von 10 bis 80 Gew.-% enthalten.

5. Verwendung nach den Ansprüchen 1 bis 4, **dadurch gekennzeichnet, dass** Emulsionen verwendet werden, die Wasser in Mengen von 20 bis 90 Gew.-% enthalten.

6. Verwendung nach den Ansprüchen 1 bis 5, **dadurch gekennzeichnet, dass** Emulsionen verwendet werden, die ein Emulgatorsystem, enthaltend hydrophile Emulgatoren mit HLB-Werten von 8 bis 18 in Kombination mit hydrophoben Coemulgatoren enthalten.

7. Verwendung nach den Ansprüchen 1 bis 6, **dadurch gekennzeichnet, dass** Emulsionen verwendet werden, deren Emulgatorsysteme Mengenverhältnisse zwischen hydrophilen Emulgatoren und Coemulgatoren von 10 : 90 bis 90 : 10 aufweisen.

8. Verwendung nach den Ansprüchen 1 bis 7, **dadurch gekennzeichnet, dass** Emulsionen verwendet werden, die Emulgatoren in Mengen von 1 bis 25 Gew.-% enthalten.

9. Verwendung nach den Ansprüchen 1 bis 8, **dadurch gekennzeichnet, dass** es sich bei den Enzymen um um Hydrolasen und/oder Acyltransferasen handelt.

10. Verwendung nach Anspruch 9, **dadurch gekennzeichnet, dass** die Hydrolasen ausgewählt sind aus der Gruppe, die gebildet wird von Esterasen, Phospholipasen, Lipasen, und Lipasen/Acyltransferasen.

11. Verwendung nach den Ansprüchen 9 und 10 **dadurch gekennzeichnet, dass** die Hydrolasen ausgewählt sind aus den Lipasen und/oder Lipasen/Acyltransferasen erhältlich aus Organismen, aus der Gruppe *Alcaligenes, Aspergillus niger, Aspergillus oryzea, Aeromonas aerophila, Bacillus species, Candida albicans, Candida antarctica (Trychosporon oryzae, Pseudozyma antarctica), Candida antarctica, Candida cylindracea, Candida glabrata, Candida maltosa, Candida parapsilosis, Candida lipolytica, Candida tropicalis, Candida viswanathii, Chromobacterium viscosum, Fusarium solani, Geotrichum candidum, Issatchenkia orientalis (Candida krusei), Kluyveromyces marxianus (C. kefyr, C. pseudotropicalis), Mucor javanicus, Penicilium camenberti, Penicilium roqueforti, Pichia guilliermondii (Candida guilliermondii), Porcine pancreas, Pseudomonas cepacia, Pseudomonas fluorescens, Rhizomucor miehei, Rhizopus arrhizus, Rhizopus oryzae, Rhizopus niveus, Rhizopus javanicus* und *Thermomyces lanugenosus* sowie deren Gemische.

12. Verwendung nach den Ansprüchen 9 bis 11 **dadurch gekennzeichnet, dass** das oder die Enzyme eingesetzt werden in einer Menge von 0,001 bis 20 Gew.-% berechnet als reines Enzym oder als Enzympräparat, bezogen auf die Gesamtmenge an eingesetzter Ölphase.

13. Verwendung nach den Ansprüchen 1 bis 12 **dadurch gekennzeichnet, dass** es sich bei den enzymatisch katalysierten Reaktionen um Hydrolyse, Veresterung oder Umesterung handelt.

14. Verwendung nach den Ansprüchen 1 bis 13, **dadurch gekennzeichnet, dass** bei der enzymatisch katalysierten Reaktion kosmetische und/oder pharmazeutische Produkte und/oder Feinchemikalien hergestellt werden.

15. Verwendung nach Anspruch 14, **dadurch gekennzeichnet, dass** es sich bei den kosmetischen und/oder pharmazeutischen Produkten und/oder den Feinchemikalien um Carotinoide, sterolhaltige Ölkomponenten und/oder Vitamin E handelt.

16. Verfahren zur enzymatisch katalysierten Veresterung, Umesterung oder Hydrolyse von Fettsäurealkylestern und/oder Triglyceriden **dadurch gekennzeichnet, dass** O/W-Emulsionen als Reaktionsmedium eingesetzt werden nach Anspruch 1 bis 8.

17. Verfahren nach Anspruch 16, **dadurch gekennzeichnet, dass** bei der enzymatisch katalysierten Reaktion kosmetische und/oder pharmazeutische Produkte und/oder Feinchemikalien hergestellt werden.

18. Verfahren nach Anspruch 16 und/oder 17, **dadurch gekennzeichnet, dass** es sich bei den kosmetischen und/oder pharmazeutischen Produkten und/oder den Feinchemikalien um Carotinoide, sterolhaltige Ölkomponenten und/oder Vitamin E handelt.

19. Verfahren nach einem der Ansprüche 16 bis18, **dadurch gekennzeichnet, dass** Enzyme nach Anspruch 9 bis 12 eingesetzt werden.

## Claims

1. Use of o/w emulsions containing at least water, emulsifiers and an oil phase as a reaction medium for enzyme-catalyzed reactions, **characterized in that** the emulsion is produced by the PIT process and has a droplet size of 50 to 400 nm.

2. Use as claimed in claim 1, **characterized in that** the oil phase contains compounds selected from the group consisting of fatty acid alkyl esters and triglycerides.

3. Use claimed in claims 1 and/or 2, **characterized in that** emulsions containing fatty acid alkyl esters corresponding to formula (I):

$R^1$-COO-$R^2$ (I)

in which $R^1$ is a $C_{6-22}$ alkyl group and $R^2$ is a $C_{1-4}$ alkyl group, are used.

**4.** Use claimed in claims 1 to 3, **characterized in that** emulsions containing the oil phase in quantities of 10 to 80% by weight are used.

**5.** Use claimed in claims 1 to 4, **characterized in that** emulsions containing water in quantities of 20 to 90% by weight are used.

**6.** Use claimed in claims 1 to 5, **characterized in that** emulsions containing hydrophilic emulsifiers with HLB values of 8 to 18 in combination with hydrophobic co-emulsifiers are used.

**7.** Use claimed in claims 1 to 6, **characterized in that** emulsions of which the emulsifier systems have quantity ratios between hydrophilic emulsifiers and co-emulsifiers of 10:90 to 90:10 are used.

**8.** Use claimed in claims 1 to 7, **characterized in that** emulsions containing emulsifiers in quantities of 1 to 25% by weight are used.

**9.** Use claimed in claims 1 to 8, **characterized in that** the enzymes are hydrolases and/or acyl transferases.

**10.** Use claimed in claim 9, **characterized in that** the hydrolases are selected from the group consisting of esterases, phospholipases, lipases and lipases/acyl transferases.

**11.** Use claimed in claims 9 and 10, **characterized in that** the hydrolases are selected from the lipases and/or lipases/acyl transferases obtainable from organisms from the group consisting of *Alcaligenes, Aspergillus niger, Aspergillus oryzea, Aeromonas aerophila, Bacillus species, Candida albicans, Candida antarctica (Trychosporon oryzae, Pseudozyma antarctica), Candida antarctica, Candida cylindracea, Candida glabrata, Candida maltosa, Candida parapsilosis, Candida lipolytica, Candida tropicalis, Candida viswanathii, Chromobacterium viscosum, Fusarium solani, Geotrichum candidum, Issatchenkia orientalis (Candida krusei), Kluyveromyces marxianus (C. kefyr, C. pseudotropicalis), Mucor javanicus, Penicilium camemberti, Penicilium roqueforti, Pichia guilliermondii (Candida guilliermondii), Porcine pancreas, Pseudomonas cepacia, Pseudomonas fluorescens, Rhizomucor miehei, Rhizopus arrhizus, Rhizopus oryzae, Rhizopus niveus, Rhizopus javanicus* and *Thermomyces lanugenosus* and mixtures thereof.

**12.** Use claimed in claims 9 to 11, **characterized in that** the enzymes are used in a quantity of 0.001 to 20% by weight, expressed as pure enzyme or as enzyme preparation, based on the total quantity of oil phase used.

**13.** Use claimed in claims 1 to 12, **characterized in that** the enzyme-catalyzed reactions are hydrolysis, esterification or transesterification reactions.

**14.** Use claimed in claims 1 to 13, **characterized in that** cosmetic and/or pharmaceutical products and/or fine chemicals are produced in the enzyme-catalyzed reaction.

**15.** Use claimed in claim 14, **characterized in that** the cosmetic and/or pharmaceutical products and/or the fine chemicals are carotinoids, sterol-containing oil components and/or vitamin E.

**16.** A process for the enzyme-catalyzed esterification, transesterification or hydrolysis of fatty acid alkyl esters and/or triglycerides, **characterized in that** o/w emulsions according to claims 1 to 8 are used as the reaction medium.

**17.** A process as claimed in claim 16, **characterized in that** cosmetic and/or pharmaceutical products and/or fine chemicals are produced in the enzyme-catalyzed reaction.

**18.** A process as claimed in claim 16 and/or 17, **characterized in that** the cosmetic and/or pharmaceutical products and/or the fine chemicals are carotinoids, sterol-containing oil components and/or vitamin E.

**19.** A process as claimed in any of claims 16 to 18, **characterized in that** enzymes according to claims 9 to 12 are used.

## Revendications

1. Utilisation d'émulsions huile-dans-l'eau en tant que milieu réactionnel pour des réactions à catalyse enzymatique, contenant au moins de l'eau, des émulsifiants, ainsi qu'une phase huileuse, **caractérisée en ce que** l'émulsion est préparée par le procédé TIP, et présente une grosseur de gouttelette de 50 à 400 nm.

2. Utilisation selon la revendication 1, **caractérisée en ce que** la phase huileuse contient des composés choisis dans le groupe formé des esters alkyliques d'acides gras et des triglycérides.

3. Utilisation selon les revendications 1 et/ou 2, **caractérisée en ce qu'**on utilise des émulsions qui contiennent des esters alkyliques d'acides gras de formule (I)

$$R^1\text{-COO-}R^2 \qquad (I)$$

dans laquelle $R^1$ est un radical alkyle ayant 6 à 22 atomes de carbone et $R^2$ est un radical alkyle ayant 1 à 4 atomes de carbone.

4. Utilisation selon les revendications 1 à 3, **caractérisée en ce qu'**on utilise des émulsions qui contiennent la phase huileuse en des quantités de 10 à 80 % en poids.

5. Utilisation selon les revendications 1 à 4, **caractérisée en ce qu'**on utilise des émulsions qui contiennent de l'eau en des quantités de 20 à 90 % en poids.

6. Utilisation selon les revendication 1 à 5, **caractérisée en ce qu'**on utilise des émulsions qui contiennent un système d'émulsifiants, lequel contient des émulsifiants hydrophiles ayant des valeurs HLB de 8 à 18 en combinaison avec des co-émulsifiants hydrophobes.

7. Utilisation selon les revendications 1 à 6, **caractérisée en ce qu'**on utilise des émulsions dont les systèmes d'émulsifiants présentent des rapports pondéraux des émulsifiants hydrophiles aux co-émulsifiants de 10:90 à 90:10.

8. Utilisation selon les revendications 1 à 7, **caractérisée en ce qu'**on utilise des émulsions qui contiennent des émulsifiants en des quantités de 1 à 25 % en poids.

9. Utilisation selon les revendications 1 à 8, **caractérisée en ce que**, pour ce qui concerne les enzymes, il s'agit d'hydrolases et/ou d'acyltransférases.

10. Utilisation selon la revendication 9, **caractérisée en ce que** les hydrolases sont choisies dans le groupe formé des estérases, des phospholipases, des lipases et des lipases/acyltransférases.

11. Utilisation selon les revendications 9 et 10, **caractérisée en ce que** les hydrolases sont choisies parmi les lipases et/ou les lipases/acyltransférases pouvant être obtenues à partir d'organismes du groupe consistant en *Alcaligenes, Aspergillus niger, Aspergillus oryzea, Aeromonas aerophila, Bacillus species, Candida albicans, Candida antarctica (Trychosporon oryzae, Pseudozyma antarctica), Candida antarctica, Candida cylindracea, Candida glabrata, Candida maltosa, Candida parapsilosis, Candida lipolytica, Candida tropicalis, Candida viswanathii, Chromobacterium viscosum, Fusarium solani, Geotrichum candidum, Issatchenkia orientalis (Candida krusei), Kluyveromyces marxianus (C. kefyr, C. pseudotropicalis), Mucor javanicus, Penicilium camenberti, Penicilium roqueforti, Pichia guilliermondii (Candida guilliermondii),* pancréas de porc, *Pseudomonas cepacia, Pseudomonas fluorescens, Rhizomucor miehei, Rhizopus arrhizus, Rhizopus oryzae, Rhizopus niveus, Rhizopus javanicus* et *Thermomyces lanugenosus,* ainsi que les mélanges de ceux-ci.

12. Utilisation selon les revendications 9 à 11, **caractérisée en ce que** la ou les enzymes sont utilisées en une quantité de 0,001 à 20 % en poids, calculée en enzymes pures ou en préparation enzymatique, par rapport à la quantité totale de la phase huileuse utilisée.

13. Utilisation selon les revendications 1 à 12, **caractérisée en ce que**, pour ce qui concerne les réactions à catalyse enzymatique, il s'agit d'une hydrolyse, d'une estérification ou d'une transestérification.

14. Utilisation selon les revendications 1 à 13, **caractérisée en ce que**, lors de la réaction à catalyse enzymatique, on

prépare des produits cosmétiques et/ou pharmaceutiques et/ou des produits de la chimie fine.

**15.** Utilisation selon la revendication 14, **caractérisée en ce que**, pour ce qui concerne les produits cosmétiques et/ou pharmaceutiques et/ou les produits de la chimie fine, il s'agit de caroténoïdes, de composants huileux contenant des stérols et/ou de vitamine E.

**16.** Procédé d'estérification, de transestérification ou d'hydrolyse à catalyse enzymatique d'esters alkyliques d'acides gras et/ou de triglycérides, **caractérisé en ce qu'**on utilise en tant que milieu réactionnel les émulsions huile-dans-l'eau selon les revendications 1 à 8.

**17.** Procédé selon la revendication 16, **caractérisé en ce que**, lors de la réaction à catalyse enzymatique, on produit des produits cosmétiques et/ou pharmaceutiques et/ou des produits de la chimie fine.

**18.** Procédé selon les revendications 16 et/ou 17, **caractérisé en ce que**, pour ce qui concerne les produits cosmétiques et/ou pharmaceutiques et/ou les produits de la chimie fine, il s'agit de caroténoïdes, de composants huileux contenant des stérols et/ou de vitamine E.

**19.** Procédé selon l'une des revendications 16 à 18, **caractérisé en ce qu'**on utilise des enzymes selon les revendications 9 à 12.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **K. Drauz ; H. Waldmann.** Enzyme Catalysis in Organic Synthesis. VCH-Verlag, 1975
- *Enzyme Microb. Technol.,* 2001, vol. 28 (1), 42-48
- **TH.Förster ; W.von Rybinski ; H.Tesmann ; A.Wadle.** Calculation of optimum emulsifier mixtures for phase inversion emulsification. *International Journal of Cosmetic Science,* 1994, vol. 16, 84-92